# EUROPEAN PATENT SPECIFICATION

(11) **EP 2 809 776 B1**
(45) Date of publication and mention of the grant of the patent: **13.04.2016**
(21) Application number: 13708533.8
(22) Date of filing: 01.02.2013
(51) Int. Cl.: C12N 9/00

(54) **PRIMER FOR AMPLIFYING GERANYL PYROPHOSPHATE SYNTHASE FROM MANGO**
PRIMER ZUR VERSTÄRKUNG VON GERANYLPYROPHOSPHATSYNTHASE AUS MANGO
AMORCE D'AMPLIFICATION DE LA GÉRANYL PYROPHOSPHATE SYNTHASE ISSUE DE LA MANGUE

(30) Priority: 03.02.2012 IN DE03022012
(43) Date of publication of application: 10.12.2014
(73) Proprietor: Council of Scientific & Industrial Research, New Delhi 110 001 (IN)
(72) Inventor: GUPTA, Vidya Shrikant, Pune 411008 (IN); KULKARNI, Ram Shridhar, Pune 411008 (IN); PANDIT, Sagar Subhash, Pune 411008 (IN); GIRI, Ashok Prabhakar, Pune 411008 (IN); PUJARI, Keshav, H, Dapoli 415712 (IN)
(74) Representative: Cabinet Plasseraud
(86) International application number: PCT/IN2013/000070
(87) International publication number: WO 2013/114405

(56) References cited:
- SAGAR S. PANDIT ET AL: "Expression profiling of various genes during the fruit development and ripening of mango", PLANT PHYSIOLOGY AND BIOCHEMISTRY, vol. 48, no. 6, 1 June 2010 (2010-06-01), pages 426-433, XP055060781, ISSN: 0981-9428, DOI: 10.1016/j.plaphy.2010.02.012 & DATABASE EMBL [Online] 2 March 2010 (2010-03-02), "Mangifera indica geranyl diphosphate synthase-like mRNA, partial sequence.", retrieved from EBI accession no. EM_STD:EU513265 Database accession no. EU513265 & DATABASE EMBL [Online] 2 March 2010 (2010-03-02), "Mangifera indica geranyl geranyl diphosphate synthase-like mRNA, partial sequence.", retrieved from EBI accession no. EM_STD:EU513267 Database accession no. EU513267

## Description

### FIELD OF THE INVENTION

The present invention relates to primer sequence for amplifying geranyl pyrophosphate synthase from mango species. The invention further relates to a nucleotide sequence encoding said amplified geranyl pyrophosphate synthase (GPPS) for enzyme production in an artificial system thus generating the desired flavor in food products.

### BACKGROUND OF THE INVENTION

Mango *(Mangifera indica* L.) represents one of the most popular tropical fruits not only in India, the country dominating global mango trade, but all over the world. Among thousands of the mango cultivars found in India, Alphonso is the most popular, mainly because of its highly attractive flavor and long shelf life. In spite of being such an ideal fruit, cultivation of Alphonso is troublesome to farmers because of various factors such as cultivation locality dependent variation in the fruit quality, especially in terms of flavor, occurrence of the physiological diseases such as spongy, alternate bearing of the fruits, etc. Alphonso fruits are highly rich in monoterpenes which contribute above 90% to the volatile blend of the ripe fruits (Pandit et al., 2009a; Pandit et al., 2009b). Further studies on the geographic variation in the mango flavor revealed that there is a variation in the levels of terpenes between cultivation localities.

Monoterpenes form one of the major classes of flavor volatiles in 'Alphonso' mango. Monoterpenes play a vital ecological role by acting as attractant of the pollinators and the seed dispersal agents, as the repellent of the herbivores and the pathogens and as an attractant of the predators of herbivores (Chen et al., 2003; Dudareva et al., 2004; Kessler and Baldwin, 2001; Pichersky and Gershenzon, 2002; Ramsewak et al., 2003). Many monoterpenes are also known to exert beneficial effects on human health by acting as antioxidant and antitumor agents (Loreto et al., 2004; Wagner and Elmadfa, 2003).

Monoterpenes also play an important role as flavor compounds of many fruits including mango giving a characteristic identity to the fruit. Being rich in the terpene flavorants, mango forms an appropriate system to study biosynthesis and regulation of monoterpenes. Studies were conducted to understand the technical composition of mango flavor indicating the dominance of monoterpenes in mango fruits.

An article titled "Softening in mango (Mangifera indica cv. Dashehari) is correlated with the expression of an early ethylene responsive, ripening related expansin gene, MiExpA1" by Vidhu Sane et.al published in Postharvest Biology and Technology, 38 (2005), 223-230, reports the isolation and characterization of an α-expansin gene, *Mi*ExpA1 that is correlated with softening in mango (*Mangifera indica* cv.Dashehari). Using degenerate primers and in combination with the 3 RACE primer, a 650 nucleotides fragment was amplified, cloned and sequenced. This sequence showed homology to other α-expansins. The full-length sequence consisted of 903 bases with an open reading frame of 777 bases that could code for a protein of 259 amino acids.

An article titled "Isolation and characterization of the MiCell gene from mango: ripening related expression and enhanced endoglucanase activity during softening" published in Plant Growth Regulation Volume 56, Number 2, 117-127, reports cloning of an endo-β-1,4-glucanase (EGase) homologue, *Mi*Cell from ripening mango (*Mangifera indica* var. Dashehari) that shows sequence similarity to higher plant EGase genes. Expression of *Mi*Cell is fruit specific and ripening related. There is a progressive increase in *Mi*Cell transcript accumulation during ripening that is correlated with increased EGase activity and associated with decrease in cellulose/hemicellulose content.

An article titled "Identification of a cDNA for the plastid-located geranylgeranyl pyrophosphate synthase from Capsicum annuum: correlative increase in enzyme activity and transcript level during fruit ripening" by M. Kuntz et al, published in The Plant Journal (1992) 2(1), 25-34, discloses that the expression of the geranylgeranyl pyrophosphate synthase gene is strongly induced during the chloroplast to chromoplast transition which occurs in ripening fruits, and is correlated with an increase in enzyme activity.

Article titled "Geranyl pyrophosphate synthase: characterization of the enzyme and evidence that this chain-length specific prenyltransferase is associated with monoterpene biosynthesis in sage (Salvia officinalis)" by Croteau R et.al. published in Arch Biochem Biophys. 1989 Jun;271(2):524-35 discloses characterization of geranyl pyrophosphate synthase from *Salvia officinalis* with respect to molecular weight, pH optimum, cation requirement, inhibitors, and kinetic parameters, and it is shown to resemble other prenyltransferases. Substrate and product specificity studies confirmed the selective synthesis of geranyl pyrophosphate by geranyl pyrophosphate synthase enzyme.

Article titled 'Expression profiling of various genes during the fruit development and ripening of mango' by Pandit et.al. published in Plant Physiology and Biochemistry 48 (2010) explores several flavor related genes along with a few associated to the physiology of developing and ripening in 'Alphonso' mango. The temporal and spatial regulation of the genes during development and ripening of 'Alphonso' mango has been analysed. Expression peaks of sHSP and MDHAR genes and the characteristic expression drop of plastid associated GPPS and MTPS genes and a drop in the correlation value at 90 days after pollination confirm that this stage as a perfect physiological maturity for harvesting. Similarly, 15 days after harvest could be marked as a perfect ripe stage by the expression peaks of FPPS, LOX, MeTr, Chitinase and UbqPL genes.

Biosynthesis of terpenes is less clearly understood with respect to geranyl pyrophosphate synthase (GPPS). It was earlier thought that plants might not have a special enzyme catalyzing the synthesis of GPP, rather small amounts of GPP produced by FPPS and GGPPS might be utilized in the biosynthesis of monoterpenes (Croteau, 1987). This was later proved to be untrue by purifying the first enzyme from the cell culture of *Lithospermum erythrorhizon* that solely synthesized GPP (Heide, 1988). This was followed by purification of similar GPPS enzymes from various other plants such as *Salvia officinalis* (Croteau and Purkett, 1989), *Pelargonium roseum* (Suga and Endo, 1991), *Vitis vinifera* (Clastre et al., 1993) and *Abies grandis* (Tholl et al., 2001). The isolation of GPPS enzyme in mint was paralleled by the discovery of the first gene for the corresponding enzyme (Burke et al., 1999).

Geranyl pyrophosphate synthases (GPPS) find utility in the flavor industry. The nucleotide sequences can be used for enzyme production in an artificial system and later this artificially synthesized enzyme can be mixed appropriately with the mango pulp, thus generating the desired flavor. These nucleotide sequences can also be used for semi-biosynthesis of flavors via various approaches such as enzyme immobilization, single cell culture, etc., as well as to improve other varieties of mango. As seen from the above disclosures, nucleotide sequences encoding Geranyl Pyrophosphate Synthases (GPPS) which play an important role in the biosynthesis of terpenes in mango is not known hitherto and there is a long standing need in the prior art for such sequences. Hence the Inventors have attempted in this research to provide artificial sequences which may be used to impart color, flavor and smell as in natural Alphonso mangoes.

### SUMMARY OF THE INVENTION

It is therefore an object of the invention to provide primers for amplifying geranyl pyrophosphate synthases from mango species useful in flavor industry, for semi-biosynthesis of flavors, for enzyme production in an artificial system as well as for improving other varieties of mango.

Accordingly, in an aspect, the invention provides primer sequences to amplify geranyl pyrophosphate synthases derived from mango having sequence selected from the group consisting of Seq ID. Nos. 2 - 13.

In another aspect, the present disclosure provides forward and reverse degenerate primers for the two geranyl pyrophosphate synthase enzymes isolated from mango.

In yet another aspect, the present disclosure provides forward and reverse gene specific primers for the two geranyl pyrophosphate synthases isolated from mango.

Yet another aspect of the current disclosure discloses primers corresponding to the terminal regions of the mRNA which are designed for the two geranyl pyrophosphate synthases isolated from mango. These terminal primers are used for the PCR amplification with mango cDNA as a template.

In yet another aspect, the present invention provides an isolated novel nucleotide sequence encoding geranyl pyrophosphate synthase from mango comprising sequence of sequence ID nos.14 and 15.

In yet another aspect, the disclosure provides a process of isolating gene sequences encoding two functional geranyl pyrophosphate synthases from mango.

In a further aspect, the disclosure provides biochemical characterization of the isolated nucleotide sequences encoding two geranyl pyrophosphate synthases.

### BRIEF DESCRIPTION OF THE DRAWINGS

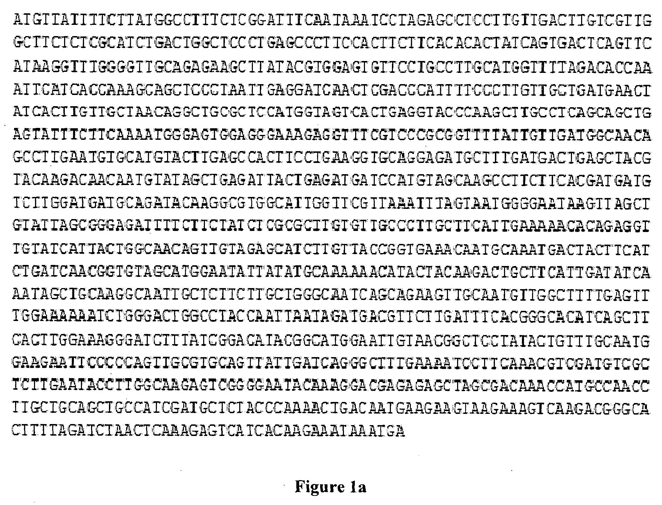

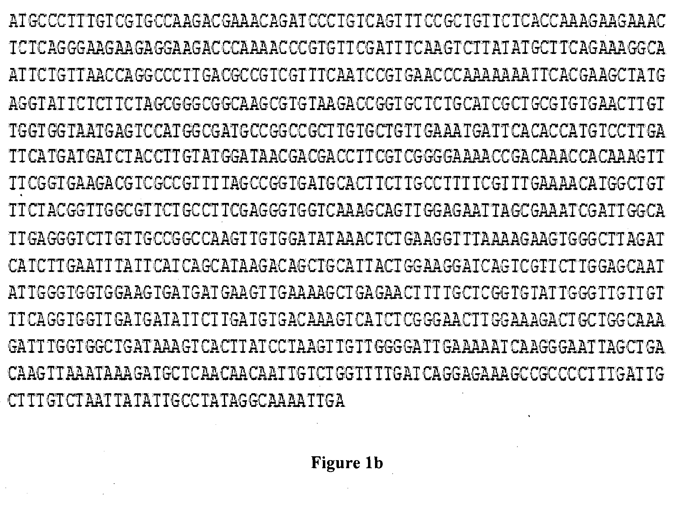

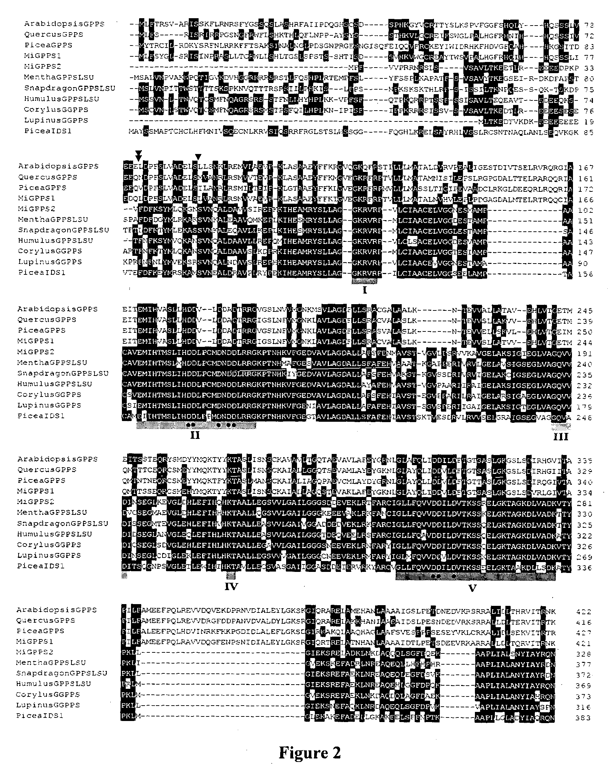

**Figure 1**(**a**)**:** Complete open reading frame encoding geranyl pyrophosphate synthase 1 (*Mi*GPPS1) isolated from mango.
**Figure 1**(**b**)**:** Comple te open reading frame encoding geranyl pyrophosphate synthase 2 (*Mi*GPPS2) isolated from mango.
**Figure 2****:** Alignment of *Mi*GPPS1 and *Mi*GPPS2 with the most similar sequences characterized from other plants. Five regions which are conserved among the prenyltransferases (I-V) are indicated by purple, coral, yellow, green and blue colour, respectively. Aspartate residues of FARM (region II) and SARM (region V) are indicated by black filled circles. The truncation site for *Mi*GPPS1 is indicated by an arrow; whereas that of *Mi*GPPS2 is shown by the arrow with two heads.
**Figure 3****:** Neighbour-Joining tree constructed using mango prenyltransferases (indicated by star symbol) and numerous other functionally characterized short-chain prenyltransferases. The numbers at the branche nodes indicate the bootstrap scores obtained using 1000 trials. Two italicized letters following the protein name indicate initials of the systematic name of the organism.
**Figure 4****:** LC-MS/MS chromatogram of the standards of GPP, FPP and GGPP (a) and of the *in vitro* assays products formed from DMAPP and IPP with the protein expressed from *Mi*GPPS1 (b), *Mi*GPPS2 (c) and the empty vector (d).
**Figure 5****:** Complementation assay for the confirmation of absence of GGPP synthase activity with *Mi*GPPS2. Functionally characterized GGPPS from *Picea abies* was used as a positive control.
**Figure 6****:** Optimum temperature, pH and MgCl₂ concentration requirement of recombinant *MI*GPPS1 (a) and *Mi*GPPS2 (b). For each enzyme and each parameter, peak area of GPP in the assay showing maximum activity was set to 1. Letters over each point indicate the significance of ANOVA (p ≤ 0.05) carried out by Fisher's LSD test independently for each of the two parameters; the values having different letters are significantly different from each other.
**Figure 7****:** Homology model of *Mi*GPPS2 generated using Mint GPPS (PDB ID: 3KRF) as a template.
   (a) Overall structure of *Mi*GPPS2 showing the 14 helices (A-N) and the five conserved regions (I-V) in colours same as those used in Figure 1.
   (b) Top-view of the model showing central reaction cavity and part of the structure harbouring the residues involved in the catalysis. Side chains of only some of the important residues in the conserved domains are indicated in colours same as those used in Figure 2. Side chains of the residues (M45 and S46) at the position corresponding to the CLD region of FPPS and GGPPS are shown in dark cyan colour.

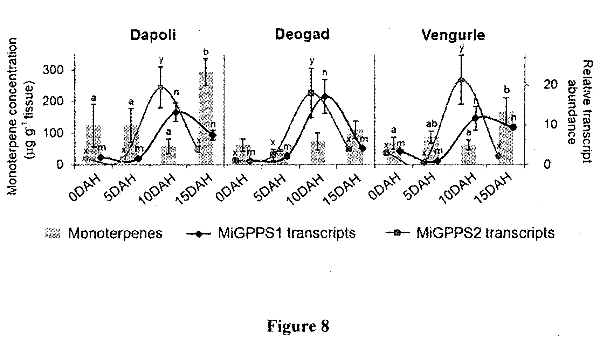

**Figure 8****:** Abundance of *Mi*GPPS1 and *Mi*GPPS2 transcripts relative to EF1α during ripening of mango fruits from three cultivation localities, Dapoli, Deogad and Vengurle, in India (DAH: days after harvest). Values presented are averages of four independent biological replicates each of which was represented by at least two technical replicates. Letters indicate the significance ofANOVA (p ≤ 0.01) for the comparison between the ripening stages for the levels of monoterpenes (a, b, etc.) and the relative transcript abundance of *Mi*GPPS1 (m, n, etc.) and *Mi*GPPS2 (x, y, etc.); the values having different letters are significantly different from each other. Letters are indicated only at the stages where the difference between the stages is significant.

### DETAILED DESCRIPTION OF THE INVENTION

The invention will now be described in detail in connection with certain preferred and optional embodiments, so that various aspects thereof may be more fully understood and appreciated.

In order to provide a clear and consistent understanding of the specification, the following definitions are provided. Unless otherwise defined herein, all technical and scientific terms used here have the same meaning as commonly understood by one skilled in the art to which the invention belongs.

'Geranyl pyrophosphate synthase' refers to an enzyme that catalyzes formation of geranyl pyrophosphate.

*Mi*GPPS1 and *Mi*GPPS2 refer to geranyl pyrophosphate synthase derived from mangifera indica (Mango) particularly. The suffix 1 & 2 are the two individual enzymes named consecutively to show they are closely related. This nomenclature is in conformance with the International protocols.

Mature raw fruits of mango used in the present invention are collected from Dapoli, Deogad and Vengurle regions of Maharashtra.

Biosynthesis of monoterpenes is thought to be localized in the plastids and it starts when the two five-carbon building blocks, Isopentenyl Pyrophosphate (IPP) and Dimethylallyl Pyrophosphate (DMAPP) are condensed into an immediate precursor of monoterpenes, geranyl pyrophosphate (GPP), by the action of GPP synthase (GPPS) (Dudareva et al., 2004). The conversion of GPP into actual monoterpenes is catalyzed by the monoterpene synthases. Formation of GPP is an important branch-point step in the biosynthesis of monoterpenes; since DMAPP and IPP are the precursors for all the terpenes. The extent of GPPS activity decides the DMAPP and IPP pool diverted towards the biosynthesis of monoterpenes which contribute above 90% to the volatile blend of the ripe mango fruit.

The key step in the biosynthesis of monoterpenes is catalyzed by geranyl pyrophosphate synthase (GPPS); which provides direct precursor-geranyl pyrophosphate (GPP)-for the biosynthesis of monoterpenes in the plants.

In an embodiment, the present invention relates to novel nucleotide sequences encoding two geranyl pyrophosphate synthase derived from mango. The nucleotide sequences encoding the two geranyl pyrophosphate synthases are useful for enzyme production in artificial system. The artificially synthesized enzyme can be mixed appropriately with the food product thus generating the desired flavor. The nucleotide sequence is also useful in the flavor industry for semi-biosynthesis of flavors via various approaches such as enzyme immobilization, single cell culture, etc., as well as for improving varieties of desired fruits and food products.

According to the present invention, the complete open reading frames encoding the two geranyl pyrophosphate synthases derived from mango are as shown in Figure **1a** and **1b****.**

The key step in the biosynthesis of monoterpenes is catalyzed by geranyl pyrophosphate synthase (GPPS); which provides direct precursor-geranyl pyrophosphate (GPP)-for the biosynthesis of monoterpenes in the plants.

Biosynthesis of monoterpenes is thought to be localized in the plastids and it starts when the two five-carbon building blocks, isopentenyl pyrophosphate (IPP) and dimethylallyl pyrophosphate (DMAPP) are condensed into an immediate precursor of monoterpenes, geranyl pyrophosphate (GPP), by the action of GPP synthase (GPPS) (Dudareva et al., 2004). The conversion of GPP into actual monoterpenes is catalyzed by the monoterpene synthases. Formation of GPP is an important branch-point step in the biosynthesis of monoterpenes ; since DMAPP and IPP are the precursors for all the terpenes. The extent of GPPS activity decides the DMAPP and IPP pool diverted towards the biosynthesis of monoterpenes which contribute above 90% to the volatile blend of the ripe mango fruit.

The isolated nucleotide sequence encoding geranyl pyrophosphate synthase from mango species having sequence selected from the group consisting of Seq. ID Nos. 14 and 15.

In another embodiment, the present invention disclose primer sequences to amplify geranyl pyrophosphate synthases derived from mango ha ving sequence selected from the group consisting of Seq Id. Nos. 2 - 13.

In another embodiment, the present inventions provides forward and reverse degenerate primers for two geranyl pyrophosphate synthases useful for amplification of the cDNA prepared from ripe fruits of mango.

The degenerate primers designed for geranyl pyrophosphate synthase 1 (GPPS1) are:
(Seq ID NO. 1) forward: 5'-TCTTGTTACNGGTGAAACCATG-3'
(Seq ID NO. 2) reverse: 5'-TYAYTTTKTTCTTGTRATGACGC -3'

The degenerate primers designed for geranyl pyrophosphate synthase 2 (GPPS2) are:
(Seq ID NO. 3) forward: 5'-TSGARATGATHCACACYATGTC -3'
(Seq ID NO. 4) reverse 1: 5'-TANGGAATRTAATTMGCYARAGC -3'
(Seq ID NO. 5) reverse 2: 5'-TTYCCWGCVGTTTTCCCCARTTC -3'

In another embodiment, the present invention provides forward and reverse gene specific primers for two geranyl pyrophosphate synthases useful for amplification of the ends of cDNA by rapid amplification of cDNA ends (RACE).

The gene specific primers designed for geranyl pyrophosphate sythase 1 (GPPS1) are:
(Seq ID NO. 6) forward 5'- AGATGACGTTCTTGATTTCACGGGC-3',
(Seq ID NO. 7) reverse 5'-CTTTGAGTTAGATCTAAAAGTGCCCG-3'

The gene specific primers designed for geranyl pyrophosphate synthase 2 (GPPS2) are:
(Seq ID NO. 8) forward 5'- ACGACCTTCGTCGGGGAAAACCG-3',
(Seq ID NO. 9) reverse 5'- GACCCTCAATGCCAATCGATTTCGC-3'

In yet another embodiment, the current invention provides primers corresponding to the terminal regions of the mRNA which are designed for two geranyl pyrophosphate synthases useful for the PCR amplification with mango cDNA as a template.

The terminal primers designed for geranyl pyrophosphate synthase 1 (GPPS1) are:
(Seq ID NO. 10) forward 5'-ATGTTATTTTCTTATGGCCTTTCTCG-3',
(Seq ID NO. 11) reverse 5'- TTTATTTCTTGTGATGACTCTTTGAG-3'

The terminal primers designed for geranyl pyrophosphate synthase 2 (GPPS2) are:
(Seq ID NO. 12) forward 5'- ATGCCCTTTGTCGTGCCAAG-3',
(Seq ID NO. 13) reverse 5'- ATTTTGCCTATAGGCAATATAATTAGAC -3'

In an embodiment, the present invention discloses the process of isolating two full-length nucleotide sequences encoding geranyl pyrophosphate synthases from ripe mangoes designated as *Mi*GPPS1 and *Mi*GPPS2 respectively. The process comprises the following steps:
i. isolating the RNA by CTAB method,
ii. treating total RNA with DNase and carrying out reverse transcription to obtain cDNA,
iii. designing degenerate primers for GPPS and GGPPS based on conserved regions in the orthologous nucleotide sequences reported in the NCBI database;
iv. amplifying cDNA of step (ii) using the degenerate primers;
v. designing gene specific primers for GPPS and GGPPS based on the sequence of the fragments obtained in step (iv);
vi. amplifying the ends of the cDNA using gene specific primers of step (v) by Rapid Amplification of cDNA Ends (RACE);
vii. design the primers corresponding to the terminal regions of mRNA by aligning the 5' and 3' RACE fragments of amplified cDNA with the orthologous nucleotide sequences; and
viii. amplifying mango cDNA using primers obtained in step (vii) by PCR (polymerase chain reaction).

Based on the conserved regions in the nucleotide sequences of geranyl pyrophosphate synthase (GPPS) reported from the other plants, primers are designed and used to amplify an interdomain fragment of these genes from Alphonso mango. The fragments obtained show high similarity to the respective genes reported from the other plants. Based on the nucleotide sequence of these fragments, gene specific primers are designed so as to have an overlapping region of 327 and 183 base pairs between 5' and 3' RACE fragments for GPPS1 and GPPS2, respectively. After each amplification step, the fragments are eluted from the agarose gel, ligated in a pGEM-T Easy vector and transformed in *E.coli* cells. Positive colonies are identified by colony PCR and the presence of desired insert is confirmed by sequencing. Sequences are aligned and analysed for the presence of uninterrupted reading frame in the MEGA 4.1 software. The full-length open reading frames are finally obtained using terminal primers based on the sequences of the RACE fragments. The fragments obtained at the end of all of the above steps encode an amino acid sequence without a stop codon.

The degenerate primers designed in step (iii) of the process of isolating two full-length nucleotide sequences encoding geranyl pyrophosphate synthases from ripe mangoes are;
1. for GPPS1:
   forward: 5'-TCTTGTTACNGGTGAAACCATG-3'
   reverse: 5'-TYAYTTTKTTCTTGTRATGACGC -3'
2. for GPPS2:
   forward: 5'-TSGARATGATHCACACYATGTC -3'
   reverse 1: 5'-TANGGAATRTAATTMGCYARAGC -3'
   reverse 2: 5'-TTYCCWGCVGTTTTCCCCARTTC -3'

The gene specific primers designed in step (v) of the process of isolating two full-length nucleotide sequences encoding geranyl pyrophosphate synthases from ripe mangoes are;
3. for GPPS 1 are:
   forward: 5'- AGATGACGTTCTTGATTTCACGGGC-3'
   reverse: 5'-CTTTGAGTTAGATCTAAAAGTGCCCG-3')
4. for GPPS2 are:
   forward: 5'- ACGACCTTCGTCGGGGAAAACCG-3'
   reverse: 5'- GACCCTCAATGCCAATCGATTTCGC-3'

The terminal primers designed in step (vii) of the process of isolating two full-length nucleotide sequences encoding geranyl pyrophosphate synthases from ripe mangoes are;
5. for GPPS1 are
   forward: 5'-ATGTTATTTTCTTATGGCCTTTCTCG-3'
   reverse: 5'- TTTATTTCTTGTGATGACTCTTTGAG-3'
6. for GPPS2 are
   forward: 5'- ATGCCCTTTGTCGTGCCAAG-3'
   reverse: 5'- ATTTTGCCTATAGGCAATATAATTAGAC -3'

The complete open reading frame (ORF) of *MiGPPS1* (Seq ID No. 14) thus obtained is 1266 base pair (bp) long, flanked by 112 base pair untranslated region (UTR) at the 5' end and 242 base pair UTR at the 3' end, encoded a protein of 421 amino acids with the predicted molecular weight of 46.2 kD and the isoelectric pH of 6.19. Similarly, the complete open reading frame (ORF) of *MiGPPS2* (Seq ID No. 15) is 987 base pair long, flanked by 343 base pair UTR at the 5' end and 144 base pair UTR at the 3' end encoded a protein of 328 amino acids with the predicted molecular weight of 35.6 kD and isoelectric point of 5.5.

In another embodiment, the present invention studies the similarity of the *in silico* translated amino acid sequences of *Mi*GPPS1 and *Mi*GPPS2 with enzymes from other plants.

The *in silico* translated *Mi*GPPS1 shows the highest sequence similarity with the GPPS from *Quercus robur* (80% identity), *Arabidopsis* (71% identity) and *Picea abies* (66% identity). The putative protein sequence of *Mi*GPPS2 on the other hand showed the highest sequence similarity to the GGPPS from *Corylus avellana* (84% identity) and *Lupinus albus* (*79%* identity), the Large Subunit (LSU) of the heterodimeric G(G)PPS from *Humulus lupulus* (81% identity) and the LSU of heterodimeric GPPS from snapdragon (78% identity). The sequence identity of the putative amino acid sequences of *Mi*GPPS1 and *MiGPPS2* with each other was 20% (Figure 2).

In another embodiment, the present invention provides a phylogenetic analysis of the deduced amino acid sequences of *Mi*GPPS1, *Mi*GPPS2 and a few functionally characterized prenyl transferases from the other organisms to understand the evolutionary relationships of *Mi*GPPS1 and *Mi*GPPS2 with the short chain prenyltransferases from the other plants. A neighbor joining tree is constructed using amino acid sequences of the genes obtained from the NCBI database. NCBI accession numbers of the sequences used are: GPPS: *Arabidopsis thaliana,* CAC16849; *Abies grandis,* AAN01134; *Quercus robur,* CAC20852; *Picea abies* (GPPS2), ACA21458; *Picea abies* (GPPS3), ACA21459; *Solanum lycopersicum,* ABB88703; *Phalaenopsis bellina,* ABV71395; *Mentha x piperita* (LSU), AAF08793; *Antirrhinum majus* (LSU), AAS82860; *Humulus lupulus* (LSU), ACQ90682; *Mentha x piperita* (SSU), AF182827; *Antirrhinum majus* (SSU), AAS82859; *Humulus lupulus* (SSU), ACQ90681; Aphid (G/FPPS), AAY33491; *Chlamydomonas reinhardtii,* XP_001691069; FPPS: *Arabidopsis thaliana,* CAB80990; *Picea abies,* ACA21460; *Panax ginseng,* AAY87903; *Artemisia spiciformis,* AAP74719; *Drosophila melanogaster,* NP_477380; *Anopheles gambiae,* XP_308653; *Homo sapiens,* NP_001995; *Escherichia coli,* NP_414955; *Micrococcus luteus,* AA25265; *Methanothermobacter marburgensis* (F/GGPPS), YP_003849447; GGPPS: *Arabidopsis thaliana,* NP_179960; *Corylus avellana,* ABW06960; *Taxus canadensis,* AAD16018; *Lupinus albus,* AAA86688; *Picea abies* (GGPPS5), ACA21461; *Picea abies* (G/GGPPS), ACZ57571; *Drosophila melanogaster,* AAC05273; *Homo sapiens,* NP_004828; *Erwinia uredovora,* P21684; *Saccharomyces cerevisiae,* NP_015256.

The analysis indicated that plant GPPS are more close to geranylgeranyl pyrophosphate synthase (GGPPS) than to Farnesyl pyrophosphate synthase (FPPS) which formed a clearly distinct cluster. GPPSs are scattered in four different clades and are accompanied by GGPPS in clade 1 (formed by gymnosperm GPPS and GGPPS) and clade 2 (formed by angiosperm GGPPS, GPPS-LSU and MiGPPS2). Clade 3 contains the angiosperm and gymnosperm GPPS including *Mi*GPPS1. Clade 4, on the other hand had the small subunit of GPPS which along with the Large Subunit (LSU) of clade 2 forms a functional heterodimeric GPPS in angiosperms.

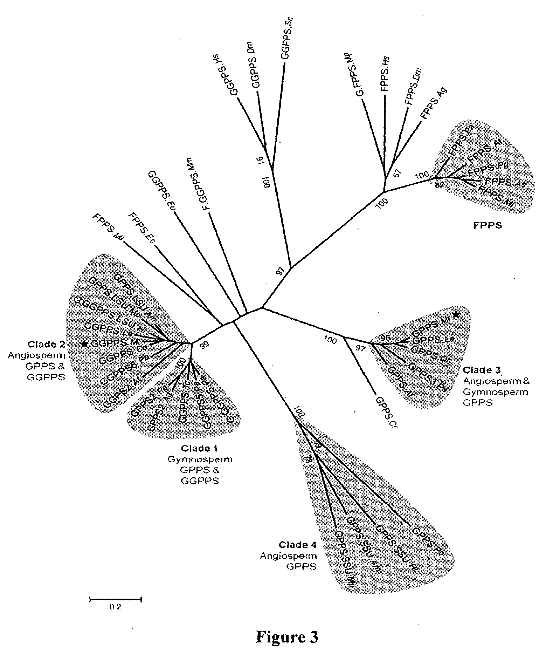

Among the four G/GGPPS clades, the enzymes of clade 1, 2 and 4 are shown to be involved in the biosynthesis of respective isopentenyl diphosphates and/or terpenes. Expression of *GPPS-SSU* of clade 4 is highly correlated with the volatiles and the expression of monoterpene synthase in *hop* and in the other plants (Wang and Dixon, 2009). The enzymes belonging to clade 1 are also shown to be involved in the terpene production induced upon methyl jasmonate treatment (Hefner et al., 1998; Schmidt and Gershenzon, 2007, 2008; Schmidt et al., 2010). Similarly, methyl jasmonate induced taxol biosynthesis in *Corylus avellana* and the spatial variation in the floral volatiles of *hop* is paralleled by the similar pattern of expression of *GGPPS* and *GPPS-LSU,* respectively, which group together in clade 2 (Wang and Dixon, 2009; Wang et al., 2010). Although the enzyme belonging to clade 3 have also been shown to have GPP synthase activity *in vitro,* no correlation between *in planta* expression of these genes and the phenotype has been found. For example, although there is an increase in the monoterpene-rich oleoresins upon methyl jasmonate treatment in spruce, the expression levels of *GPPS3* belonging to clade 3 are unaltered (Schmidt and Gershenzon, 2007). Similarly, silencing of *GPPS* in tomato results in dwarfed plants, which is further shown to be because of reduced gibberellin content (van Schie et al., 2007). These studies point towards involvement of members of clade 3 in the functions in addition to/other than monoterpene biosynthesis. Clustering of *MiGPPS1* with clade 3 enzymes (Fig. 3) suggests additional role of *Mi*GPPS1 in mango.

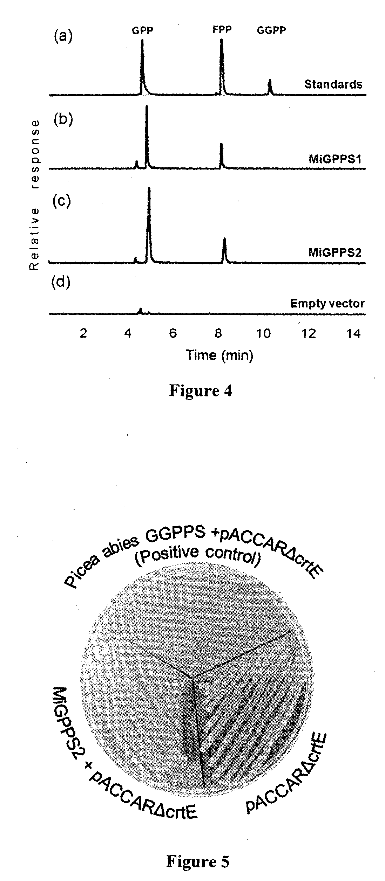

In another embodiment, the present disclosure provides *in vitro* assay to get functional insights into the recombinant proteins of *Mi*GPPS1 and *Mi*GPPS2. Accordingly, the recombinant proteins are incubated with substrates isopentenyl pyrophosphate (IPP) and dimethylallyl pyrophosphate (DMAPP) and the products are analysed by LC-MS/MS. None of the isopentenyl diphosphate products are detected in the assays with the soluble fraction of the full-length and truncated version of *Mi*GPPS1 and *Mi*GPPS2. For *Mi*GPPS1 when sorbitol and betaine were included in the LB media, the soluble fraction of the truncated version showed the formation of GPP along with about 7-12% (of the total) FPP at the optimum conditions. The same medium was used for truncated version of *Mi*GPPS2 and surprisingly, instead of GGPP, the purified protein formed E-GPP as a main product with about 8-16% *E*,*E*-FPP, at the optimum conditions. None of the isopentenyl diphosphate products were detected with the full-length versions of *Mi*GPPS1 and *Mi*GPPS2 carrying the 5' segment corresponding to the putative signal peptides. The assays with protein expressed from an empty vector, as well as the substrate- and enzyme-blank assays did not show the presence of any of the isopentenyl diphosphate products confirming the *in vitro* activities of the recombinant proteins (Fig 4).

In another embodiment, the present disclosure provides a complementation assay for *Mi*GPPS2 to confirm the absence of geranylgeranyl pyrophosphate (GGPP) synthase activity with *Mi*GPPS2.

Accordingly, the plasmid pACCARΔcrtE is constructed by cloning the *Erwinia uredovora* genes of caratogenesis (*crt*) cluster except *GGPPS* (Sandmann et al., 1993). When pACCATΔcrtE is complemented by the functional GGPPS, yellow colored zeaxanthin diglucoside pigment is formed. The putative *GGPPS* coding sequence from mango analyzed in this manner does not produce yellow-coloured colonies confirming the absence of GGPP synthase activity with the putative GGPPS from mango. This finding along with the GPP synthase activity detected in *in vitro* assays results in nomenclature of the putative GGPPS as *MiGPPS2.*

In another embodiment, the present disclosure provides enzymatic assays to determine the optimum biochemical requirements of *Mi*GPPS1 and *Mi*GPPS2.

Accordingly, the activity of *Mi*GPPS1 and *Mi*GPPS2 is measured at varying temperature, MgCl₂ concentration and pH. In contrast to the low sequence identity between *Mi*GPPS1 and *Mi*GPPS2, biochemical properties of these two enzymes are much similar. The relatively higher optimum temperature (40 °C) of *Mi*GPPS1 and *Mi*GPPS2 can be attributed to the higher temperature observed in the ripening fruits of mangoes.

During the enzymatic reaction of prenyltransferases, binding of divalent metal ions such as Mg²⁺ to the allylic substrate is necessary for the dissociation of pyrophosphate moiety of the allylic molecule. Mg²⁺ is also required for the binding of the substrate molecules to the enzyme (King and Rilling, 1977). These facts clearly explain the absence of the activity of *Mi*GPPS1 and *Mi*GPPS2 in the absence of MgCl₂. Many of the GPPSs reported till now are at least partially active with Mn²⁺ as a cofactor instead of Mg²⁺ (Croteau and Purkett, 1989; Tholl et al., 2001) ; whereas, some of the GPPSs prefer Mn²⁺ over Mg²⁺ (Clastre et al., 1993; Suga and Endo, 1991). On the contrary, none of the *Mi*GPPS1 and the *Mi*GPPS2 show any activity when Mn²⁺ is used as a divalent metal ion. Since mango fruits contain more than 600 fold higher concentration of magnesium as compared to manganese (Malik et al., 2004), the lack of activity with Mn²⁺ can be explained as an adaptation of *Mi*GPPSs to the higher concentration of Mg²⁺ in the fruits. The activity profile with the varying Mg²⁺concentration, the optimum Mg²⁺ concentration of 6mM and the pH optima between 7 and 8 of *MiGPPS1* and *MiGPPS2* is quite similar to the GPPS reported from *Abies grandis* (Tholl et al., 2001).

In another embodiment, the present invention provides a homology based model of *Mi*GPPS2 using the large sub unit (LSU) of mint GPPS as template. LSU of mint shows 78% sequence identity with *Mi*GPPS2.

In *Mi*GPPS2, the residues in the region corresponding to the chain-length determining (CLD) stretch are smaller (methionine and serine) and hence, in the modeled structure of *Mi*GPPS2, none of these residues appear to have their side chains protruding into the activity cavity. This underlines the possibility of regulation of the product size of *Mi*GPPS2 by some other residues. The mutagenesis studies on FPPS and GGPPS have indicated that alterations of amino acids other than those of the active site and the CLD region can also result in the different products (Hemmi et al., 2003; Hirooka et al., 2000; Kawasaki et al., 2003; Lee et al., 2004). In addition, it is shown that out of merely eight relevant amino acid differences between the GPPS and GGPPS reported from grand fir, only one lie in the conventional CLD region (Burke et al., 2004). Conversion of each of this residue in GPPS, individually to the corresponding residue in GGPPS does not affect the product distribution indicating that more than one amino acid is involved in the chain-length determination. Although no obvious amino acid differences in the alignment between and in the structure of *Mi*GPPS2, mint GPPS-LSU and *Sinapis* GGPPS (Kloer et al., 2006) could be spotted, an unexpected activity of the *Mi*GPPS2 as a GPP synthase instead of GGPP synthase might be due to the cumulative effect of several minor amino acid differences distant from the active site and the conventional CLD region.

In contrast to GGPPS and FPPS, which have been well understood for the chain-length determining mechanism, not much is known about the means by which GPPS restrict its products length to C10. In case of GGPPS and FPPS, it has been shown that the two amino acids at the -5 and -4 position before FARM form the chain-length determining region. If the residues in this region are bulky and aromatic like phenylalanine and tyrosine, their side-chains protrude into the reaction cavity which blocks the further chain elongation leading to formation of shorter chain products such as FPP. In case of almost all GPPS reported till now, the corresponding amino acids are much smaller, such as alanine/methionine and serine. The chain-length determination mechanism of GPPS thus appears to be different than that of FPPS and GGPPS.

In case of the heterodimeric GPPS the unawareness about the product chain-length determining mechanism is further aggravated by the fact that the catalytic large sub unit (LSU) shares very high sequence homology with the GGPPS. More importantly, the residues in the chain-length determining (CLD) region of GGPPS are also conserved among the GPPS-LSU and still there is a difference of C10 between the products of these two enzymes (Burke et al., 1999). Such high sequence similarity of GPPS-LSU with GGPPS is compensated by the interactions between LSU and SSU, which results in the remodeling of the reaction cavity and restriction of the product length to GPP (Burke and Croteau, 2002; Chang et al., 2010; Orlova et al., 2009). *Mi*GPPS2 is also highly similar to GGPPS and GPPS-LSU, nonetheless, it does not produce GGPP but synthesizes GPP and small amounts of FPP in the absence of any regulatory small sub unit (SSU).

In another embodiment, the present disclosure profiles the transcripts of *Mi*GPPS1 and *Mi*GPPS2 through the ripening stages of mango from three cultivation localities in Maharashtra; Dapoli, Deogad and Vengurle.

Accordingly, in the ripening fruits of mango, the maximum expression of *MiGPPS1* and *MiGPPS2* is observed to be at 10 days after harvest (DAH), while the concentration of monoterpenes is observed to be the highest at 15 DAH (Pandit et al., 2009b) indicating the preparative role of *MiGPPS1* and *MiGPPS2* for the highest production of monoterpenes at 15 DAH.

### Industrial advantages

Monoterpenes are the most important components of the terpene class of the represent flavor and fragrance chemicals. The coding sequences of the enzyme, geranyl diphosphate synthase, characterized in this study can be used for biotechnological production of the recombinant enzyme which can be further used for the production of monoterpenes. The degenerate primers described here have been designed by homology-based approach based on the putative gene sequences reported from the other plants. These primers can thus be used for isolating similar genes from the other plants also. Similar work is being attempted by the Inventors in case of Alphonso mango as well as other economically important fruits and crops.

Particularly for this invention the nucleotide sequences encoding the two geranyl pyrophosphate synthases of the present invention are useful for enzyme production in artificial system. This artificially synthesized enzyme can be mixed appropriately with the food product including mango pulp, thus generating the desired flavor. The nucleotide sequence is also useful in the flavor industry for semi-biosynthesis of flavors via various approaches such as enzyme immobilization, single cell culture, etc., as well as for improving other varieties of mango.

### References cited in the specification

Burke, C., Croteau, R., 2002. Geranyl diphosphate synthase from Abies grandis: cDNA isolation, functional expression, and characterization. Archives of Biochemistry and Biophysics 405, 130-136.
Burke, C., Klettke, K., Croteau, R., 2004. Heteromeric geranyl diphosphate synthase from mint: construction of a functional fusion protein and inhibition by bisphosphonate substrate analogs. Archives of Biochemistry and Biophysics 422, 52-60.
Burke, C. C., Wildung, M. R., Croteau, R., 1999. Geranyl diphosphate synthase: Cloning, expression, and characterization of this prenyltransferase as a heterodimer. Proceedings of the National Academy of Sciences of the United States of America 96, 13062-13067.
Chang, T. H., Hsieh, F. L., Ko, T. P., Teng, K. H., Liang, P. H., Wang, A. H. J., 2010. Structure of a Heterotetrameric Geranyl Pyrophosphate Synthase from Mint (Mentha piperita) Reveals Intersubunit Regulation. Plant Cell 22, 454-467.
Chen, F., Tholl, D., D'Auria, J. C., Farooq, A., Pichersky, E., Gershenzon, J., 2003. Biosynthesis and emission of terpenoid volatiles from Arabidopsis flowers. Plant Cell 15,481-494.
Chourasia, A., Sane, V. A., Singh, R. K., Nath, P., 2008. Isolation and characterization of the MiCell gene from mango: ripening related expression and enhanced endoglucanase activity during softening. Plant Growth Regulation 56, 117-127.

### EXAMPLES

The following examples are given by way of illustration and therefore should not be construed to limit the scope of the present invention.

### EXAMPLE 1

### isolation of full-length MiGPPS1 and MiGPPS2

### Plant material:

Mature raw fruits of mango were collected from the orchards of Konkan Krishi Vidyapeeth at Dapoli (N17°45' E73°11') and Deogad (N16°31' E73°20') and from a private orchard at Vengurle (N15°51' E73°39'). For each of the three localities, fruits were collected from four plants. After harvesting, fruits were put in the hay, carried to the laboratory and allowed to ripe at ambient temperature. At the interval of every five days, fruits were peeled, pulp was immediately frozen in the liquid nitrogen and stored at -80 °C until use. Thus, the experimental tissues of four ripening stages: 0, 5, 10 and 15 DAH (days after harvest) were obtained from each of the three localities.

### EXAMPLE 2

### RNA isolation and cDNA synthesis

RNA was isolated by modified CTAB method as described earlier (Pandit et al., 2007). After treating total RNA with DNase, reverse transcription was carried out over 1µg of total RNA using Enhanced Avian RT First Strand Synthesis Kit (Sigma, St. Louis, MO, USA).

Based on the conserved regions in the orthologous nucleotide sequences reported in the NCBI database, degenerate primers were designed for GPPS (for 1: 5'-TCTTGTTACNGGTGAAACCATG-3' and rev: 5'-TYAYTTTKTTCTTGTRATGACGC - 3') and GGPPS (for: 5'-TSGARATGATHCACACYATGTC -3', rev 1: 5'-TANGGAATRTAATTMGCYARAGC -3', rev2: 5'- TTYCCWGCVGTTTTCCCCARTTC -3'). These primers were used for amplification of the cDNA prepared from the ripe fruits of mango. The gene specific primers for GPPS (for5'-AGATGACGTTCTTGATTTCACGGGC-3' ,rev5'-CTTTGAGTTAGATCTAAAAGTGCCCG-3') and GGPPS (for 5'-ACGACCTTCGTCGGGGAAAACCG-3', rev 5'-GACCCTCAATGCCAATCGATTTCGC-3') designed based on the sequence of the fragments obtained were used for amplification of the ends of the cDNA by rapid amplification of cDNA ends (RACE) using the SMART™ RACE cDNA Amplification Kit (Clontech, Palo Alto, CA, USA). Based on the alignments of the 5' and 3' RACE fragments with the orthologous sequences reported on NCBI database, primers corresponding to the terminal regions of the mRNA were designed for GPPS (for 5'-ATGTTATTTTCTTATGGCCTTTCTCG-3' ,rev5'-TTTATTTCTTGTGATGACTCTTTGAG -3') and GGPPS (for 5'-ATGCCCTTTGTCGTGCCAAG -3', rev 5'- ATTTTGCCTATAGGCAATATAATTAGAC -3') and were used for the PCR with mango cDNA as a template. After each step of PCR mentioned above, the fragments were eluted from the agarose gel, ligated in pGEM-T Easy vector (Promega, Madison, WI, USA) and the ligation reactions were transformed in E. coli cells (Top10). Positive colonies were identified by colony PCR and the presence of desired insert was confirmed by sequencing. Sequences were aligned and analysed for the presence of uninterrupted reading frame in the MEGA 4.1 software.

### EXAMPLE 3

### Phylogenetic analysis

To understand the evolutionary relationships of *Mi*GPPS1 and *Mi*GPPS2 with the short chain prenyltransferases from the other plants, phylogenetic analysis was carried out using the deduced amino acid sequences of *Mi*GPPS1, *Mi*GPPS2 and a few functionally characterized prenyl transferases from the other organisms. Amino acid sequences of the genes were obtained from the NCBI database and used for constructing a neighbour joining tree using MEGA software (Version 5.02). The percent bootstrap values were obtained from 1000 replicates.

The analysis indicated that plant GPPS are more close to GGPPS than to FPPS which formed a clearly distinct cluster. GPPSs were scattered in four different clades and were accompanied by GGPPS in clade 1 (formed by gymnosperm GPPS and GGPPS) and clade 2 (formed by angiosperm GGPPS, GPPS-LSU and MiGGPPS (MiGPPS2)). Clade 3 contained the angiosperm and gymnosperm GPPS including MiGPPS (MiGPPS1). Clade 4, on the other hand had the small subunit of GPPS which along with the LSU of clade 2 forms a functional heterodimeric GPPS in angiosperms (Fig. 3).

### Expression cloning and recombinant expression of MiGPPS1 and MiGPPS2 in E.coli

Full length sequence of *Mi*GPPS1 and *Mi*GPPS2 were amplified from the cDNA prepared from ripe fruits, using Expand High Fidelity PCR System (La Roche, Basel, Switzerland) with the terminal primers described above and the resulting fragment was cloned in the pEXP5-CT/TOPO expression vector (Invitrogen, Carlsbad, CA, USA). Ligation reaction was transformed in the *E. coli* strain TOP10F' (Invitrogen) and the transformants were selected on the LB-agar media containing 100 µg/ml carbanecillin. After confirming the correct orientation of the insert and the presence of an un-interrupted reading frame, the recombinant plasmids of *Mi*GPPS1 and *Mi*GPPS2 were transformed in the BL21(DE3)pLysS Rosetta (Novagen, Madison, WI, USA) and BL21(DE3) Star (Invitrogen) cells, respectively. LB media containing 1M sorbitol and 2.5mM betaine was used for the expression of the recombinant proteins. 5ml of starter culture grown at 18 °C for 48 hrs was used as inoculum for the expression in 100ml media with the Overnight Express Autoinduction System 1 (Novagen, Madison, WI, USA). Cultures were grown for 24 hrs at 18 °C and the pellet obtained after centrifugation was resuspended in the buffer containing 25mM MOPSO (pH 7.2), 10mM MgCl2 and 10% (v/v) glycerol and lysed by sonication. The (his)6-tagged recombinant protein was purified by passing the cleared lysate through Ni-NTA resin (Novagen, Madison, WI, USA) following the manufacturer's instructions. Elution was carried out with the buffer containing 250mM imidazole, 25mM MOPSO (pH 7.2), 10mM MgCl₂ and 10% (v/v) glycerol. Both crude lysate and the purified protein were checked for the presence of the recombinant protein by SDS-PAGE.

In agreement with the prediction that none of these proteins would be soluble in *E. coli* upon overexpression, no much protein was obtained in the soluble fraction for *Mi*GPPS (*Mi*GPPS1) and *Mi*GGPPS (*Mi*GPPS2) during the initial experiments. Out of the several modifications made to the growth media for obtaining soluble protein, inclusion of 1 M sorbitol and 2.5 mM betaine in the LB media yielded active protein in the soluble fraction.

### EXAMPLE 4

### Assay for the enzymatic activity

In vitro assay for determining the activity of *Mi*GPPS1 and *Mi*GPPS2 were carried out in the final volume of 200 µl containing appropriate amount of enzyme, 25 mM Mopso (pH 7.0), 2 mM DTT, 10 mM MgCl₂, 10% (v/v) glycerol and 67 µl of each DMAPP and IPP. For the optimum pH determination of the recombinant enzymes, the assays were performed in 25 mM MOPSO (pH 6 and 6.5), 25 mM HEPES (pH 7 and 7.5) or 25 mM tris (pH 8-9) containing the other required components as mentioned above. The assays carried out for determining the optimum Mg²⁺ concentration, contained varied concentration MgCl₂ along with the other required components as mentioned above. After overnight incubation, the assay reactions were washed with equal volume of chloroform for removing the proteins and the aqueous phase was directly used for LC-MS/MS analysis.

### EXAMPLE 5

### LC-MS/MS analysis

Analysis of isoprenoid pyrophosphates was performed on an Agilent 1200 HPLC system (Agilent Technologies, Santa Clara, CA, USA) coupled to an API 3200 triple quadrupole mass spectrometer (Applied Biosystems, Forster City, CA, USA). The used column was an Agilent ZORBAX Extended C-18; 1.8 µm, 50 x 4.6 mm (Agilent Technologies). Mobile phase consisted of 5 mM Ammonium bicarbonate in water as solvent A and acetonitrile as solvent B, flow rate was set at 0.8 ml/min and column temperature at 20 °C. Separation was achieved using a gradient starting at 0% B increasing to 10% B in 2 min, 64% B in 12 min and 100% B in 2 min keeping it at 100% B for 1 min followed by a change to 0% B in 1 min and keeping it there for 5 min before the next injection. Injection volume for samples and standards was 10 µl. Mass spectrometer was used in negative electrospray ionization mode. Optimal settings were determined using standards purchased from Sigma-Aldrich. Ion source gas 1 and 2 were set at 60 and 70 psi having a temperature of 700 °C, curtain gas was set at 30 psi and collision gas at 7 psi. Ion spray voltage was maintained at -4200 V. Monitored MRM transitions were m/z 312.9/79 for GPP, m/z 380.9/79 for FPP and m/z 449/79 for GGPP. Data analysis was performed using Analyst Software 1.5 Build 3385 (Applied Biosystems).

None of the isopentenyl diphosphate products were detected in the assays with the soluble fraction of the full-length and the truncated versions of MiGPPS (MiGPPS1) and MiGGPPS (MiGPPS2) obtained with the LB media. For MiGPPS (MiGPPS1), when sorbitol and betaine were included in the LB media, the soluble fraction of the truncated version showed the formation of GPP along with about 7-12% (of the total) FPP at the optimum conditions (Fig. 4). The same medium was used for truncated version of MiGGPPS (MiGPPS2) and the purified protein, surprisingly, did not show any GGPP forming activity, rather GPP was detected as a main product with about 8-16% (of the total) FPP at the optimum conditions. None of the isopentenyl diphosphate products were detected with the full-length versions of MiGPPS (MiGPPS1) and MiGGPPS (MiGPPS2) and with the protein expressed from an empty vector, confirming the *in vitro* activities of the recombinant proteins.

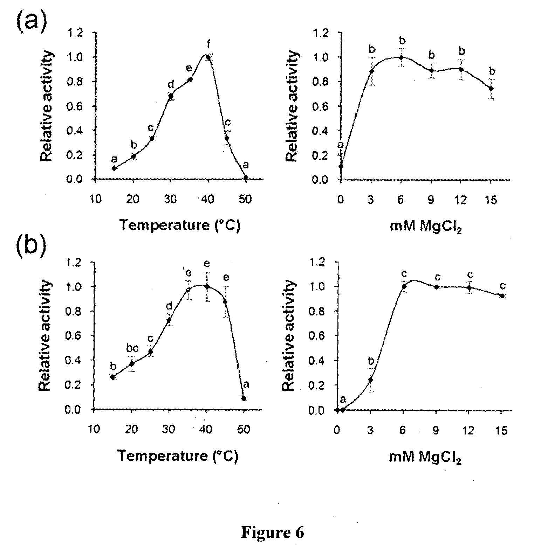

To know the optimum biochemical requirements of *Mi*GPPS1 and *Mi*GPPS2, *in vitro* assays were carried out by measuring the activity at varying temperature, MgCl₂ concentration and pH. Both the enzymes required the temperature of 40 °C for the optimum activity and more than 60% of the optimum activity was retained at 30 and 35 °C. The activity sharply reduced above the temperature of 40 °C. The MgCl₂ concentration of 6mM was required for both of the enzymes for exhibiting the maximum activity. *Mi*GPPS1 showed more than 80% of the optimum activity at the MgCl₂ concentration of 3-15mM; similar extent of activity was exhibited by *Mi*GPPS2 between 6 and 15mM MgCl₂. None of these enzymes showed any activity in the absence of Mg²⁺ or in the presence of other divalent metal ions such as Mn²⁺, Zn²⁺ or Ca²⁺. Both *Mi*GPPS1 and *Mi*GPPS2 were optimally active between pH 7 and 8 (Fig. 6).

### EXAMPLE 6

### Complementation assay

*Mi*GPPS2 showed the higher sequence similarity with the GGPPS than GPPS reported from the other plants but no GGPP was detected in the *in vitro* assays. To confirm the absence of GGPP synthase activity with the *Mi*GPPS2, complementation assay was carried out by co-transforming *Mi*GPPS2 with pACCARΔcrtE. PaIDS5 from *Picea abies,* which has been shown to be a functional GGPPS, was used as a positive control. Plasmids in the combination of PaIDS5+ pACCARΔcrtE, *Mi*GPPS2 + pACCARΔcrtE, and pACCARΔcrtE alone, were transformed in BL21(DE3)Star cells (Invitrogen). The transformants were selected on LB agar media containing 100µg/ ml carbanecillin and 50µg/ ml chloramphenicol and were allowed to grow at 28 °C for 2 days.

The colonies obtained with the co-transformation of *Pa*IDS5 with pACCARΔcrtE showed the formation of yellow pigments; whereas, no yellow colored colonies were seen with *Mi*GPPS2- pACCARΔcrtE cotransformation (Fig. 5). This confirmed that *Mi*GPPS2 does not exhibit GGPP forming activity *in vivo* also.

### EXAMPLE 7

### Homology modeling

Three-dimensional structure of the putative *Mi*GPPS2 was determined on CPH models 3.0 server (Nielsen et al., 2010). GPPS-LSU of Mint (PDB ID: 3KRF) (Chang et al., 2010) which shows 78% sequence identity with *Mi*GPPS2 was used as a template. Ramchandran plot assessment of the structure was carried out on RAMPAGE server (Lovell et al., 2003). Further quality parameters of the generated model were assessed on a web-based program, ProSA (Sippl, 1993; Wiederstein and Sippl, 2007). The final structure was visualized in the program UCSF Chimera, production version 1.5.

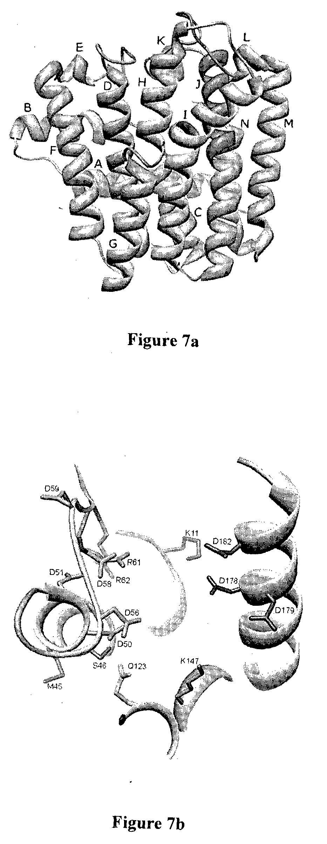

Assessment of the structure by a Ramachandran plot showed the presence of 98.3% residues in the favoured region and 1.7% residues in the allowed region. The structure was further evaluated by Protein Structure Analysis tool yielding the Z-score of -8.18 and negative energy values for all the residues in the energy plot. The root mean square deviation (RMSD) between the modeled structure of *Mi*GPPS2 and the template (LSU of mint GPPS) was 0.55 Å. All of these assessments indicated the good quality of the model generated for *Mi*GPPS2. The structure of *Mi*GPPS2 composed of 14 helices (A to N) that surrounded the central reaction cavity (Fig. 7a and 7b). Last two residues of helix D and a loop between helix D and E had the first aspartate rich motif (FARM); whereas, helix J had the second aspartate rich motif SARM. Side chains of all the aspartate residues of FARM and SARM, which were present facing each other in the central reaction cavity, and those of the two arginine residues proceeding FARM, pointed towards the central activity cavity suggesting their role in the catalytic activity.

### EXAMPLE 8

### qRT-PCR

Quantitative PCR was performed with Brilliant SYBR Green QPCR Master Mix (Stratagene) with elongation factor 1α (EF1α) as an internal control. Primers used for amplifying a fragment of *Mi*GPPS1 were: for 5'-AGGCTGCGCTCCATGGTAGTCA- 3' and rev 5'-ACCGTGGGACGAAACCTCTTTCC-3'; whereas, those for *Mi*GPPS2 were: for 5'-GACTGCTGGCAAAGATTTGGTGGCT- 3' and rev 5'-GGCGGCTTTCTCCTGATCAAAACCA -3'. For EF1α the primers used were same as described earlier (Pandit et al., 2010). At least three amplicons per gene were cloned and sequenced to confirm the primer specificity. Transcript abundance was quantified with a Mx3000P Real Time PCR Thermocycler (Stratagene, La Jolla, CA, USA) using a program with 45 cycles of 95 °C for 30 s, 63 °C for 30 s and 72 °C for 30 s, followed by a melting curve analysis of transcripts. For both the genes, the relative transcript abundance for the raw stage (0 DAH) was considered 1 and the fold difference for the rest of the tissues was calculated. Each measurement was repeated with four independent biological replicates, each of which was represented by at least two technical replicates.

### 1. MiGPPS1

For all of the three localities, Dapoli, Deogad and Vengurle, the fruits of 10 days after harvest (10 DAH) showed the highest expression (Fig. 8). The transcript levels were about 3.4 (Vengurle) to 17 (Deogad) fold higher in 10 DAH stage than the raw (0 DAH) stage. Although the expression was higher in the ripe (15 DAH) stage as compared to the raw stages, there was a slight reduction in the expression during the transition from 10 DAH to 15 DAH stage. No uniform difference between the localities through the ripening stages could be seen for the expression levels of *MiGPPS1.* Deogad fruits had the higher expression levels in 5 DAH and 10 DAH stage and the lower expression levels in the 0 DAH and 15 DAH stage as compared to Dapoli and Vengurle; however, this difference was only 1.2 to 3 folds. Expression of *MiGPPS1* was also assessed in the raw and ripe exocarp of the fruits from Deogad; ripe skin had about 5 fold higher transcripts as compared to the raw skin. The transcript levels were 3.9 and 4.4 folds higher in the exocarp as compared to the mesocarp for the raw and ripe stages, respectively.

### 2. MiGPPS2

For all of the three localities, Dapoli, Deogad and Vengurle, the fruits of 10 days after harvest (10 DAH) showed the highest expression (Fig. 8). This transcript level was about 7.2 (Vengurle) to 18 (Deogad) fold higher in 10 DAH stage than the raw (0 DAH) stage. There was a 4.5 (Deogad) to 9.5 (Vengurle) fold reduction in the expression during the transition from 10 DAH to 15 DAH stage. When localities were compared to each other, Deogad fruits had higher expression levels in 5 DAH and 15 DAH stage and lower expression levels in the 0 DAH and 10 DAH stage as compared to Dapoli and Vengurle.

### EXAMPLE 9

### Statistical analysis

Comparison between ripening stages for the levels of monoterpenes and the relative transcript abundance of *Mi*GPPS1 and *Mi*GPPS2 was carried out by ANOVA by Fisher's LSD test at p < 0.05 with the aid of StatView software, version 5.0 (SAS Institute Inc., Cary, NC, USA).

## Claims

1. A primer sequence for amplifying geranyl pyrophosphate synthase having sequence selected from the group consisting of Seq Id. Nos. 2 - 13.

2. An isolated novel nucleotide sequence encoding geranyl pyrophosphate synthase from mango comprising sequence ID nos. 14 and 15.

3. Use of geranyl pyrophosphate synthase gene having sequence selected from the group consisting of Seq Id nos. 14 and 15 obtained by using primer sequence as claimed in claim 1 for enzyme production in an artificial system.

4. Use of geranyl pyrophosphate synthase gene having sequence selected from the group consisting of Seq Id nos. 14 and 15 obtained by using primer sequence as claimed in claim 1 for semi-biosynthesis of flavors.

5. Use of geranyl pyrophosphate synthase gene having sequence selected from the group consisting of Seq Id nos. 14 and 15 obtained by using primer sequence as claimed in claim 1 for improving mango varieties.

## Patentansprüche

1. Primersequenz zum Amplifizieren einer Geranylpyrophosphat-Synthase mit einer Sequenz ausgewählt aus der Gruppe bestehend aus Seq Id. Nos. 2-13.

2. Isolierte, neue Nukleotidsequenz, die für eine Geranylpyrophosphat-Synthase aus Mango kodiert, umfassend Sequenz ID Nos. 14 und 15.

3. Verwendung eines Geranylpyrophosphat-Synthase-Gens mit einer Sequenz ausgewählt aus der Gruppe bestehend aus Seq Id. Nos. 14 und 15, erhalten durch Verwendung der in Anspruch 1 beanspruchten Primersequenz, zur Enzymherstellung in einem künstlichen System.

4. Verwendung eines Geranylpyrophosphat-Synthase-Gens mit einer Sequenz ausgewählt aus der Gruppe bestehend aus Seq Id. Nos. 14 und 15, erhalten durch Verwendung der in Anspruch 1 beanspruchten Primersequenz, zur Semi-Biosynthese von Geschmacksstoffen.

5. Verwendung eines Geranylpyrophosphat-Synthase-Gens mit einer Sequenz ausgewählt aus der Gruppe bestehend aus Seq Id. Nos. 14 und 15, erhalten durch Verwendung der in Anspruch 1 beanspruchten Primersequenz, zur Verbesserung von Mango-Sorten.

## Revendications

1. Séquence d'amorce pour l'amplification de la géranyl-pyrophosphate synthase ayant une séquence choisie dans le groupe constitué de SEQ ID NO : 2 à 13.

2. Séquence nucléotidique nouvelle isolée codant pour la géranyl-pyrophosphate synthase issue de la mangue comprenant les séquences SEQ ID NO : 14 et 15.

3. Utilisation du gène de la géranyl-pyrophosphate synthase ayant une séquence choisie dans le groupe constitué de SEQ ID NO : 14 et 15 obtenue en utilisant la séquence d'amorce telle que revendiquée dans la revendication 1 pour la production d'enzymes dans un système artificiel.

4. Utilisation du gène de la géranyl-pyrophosphate synthase ayant une séquence choisie dans le groupe constitué de SEQ ID NO : 14 et 15 obtenue en utilisant la séquence d'amorce telle que revendiquée dans la revendication 1 pour la semi-biosynthèse d'arômes.

5. Utilisation du gène de la géranyl-pyrophosphate synthase ayant une séquence choisie dans le groupe constitué de SEQ ID NO : 14 et 15 obtenue en utilisant la séquence d'amorce telle que revendiquée dans la revendication 1 pour l'amélioration des variétés de mangue.


### SEQUENCE LISTING

<110> CSIR, IN
   <120> PRIMER FOR AMPLIFYING GERANYL PYROPHOSPHATE SYNTHASE
   <130> 0302DEL2012
   <160> 15
   <170> PatentIn version 3.5
<210> 1
   <211> 22
   <212> DNA
   <213> ARTIFICIAL SEQUENCE
   <220>
   <223> Degenerate Forward Primer- GPPS 1
   <220>
   <221> misc_feature
   <222> (10)..(10)
   <223> n is a, c, g, or t
<400> 1

   tcttgttacn ggtgaaacca tg 22
<210> 2
   <211> 23
   <212> DNA
   <213> ARTIFICIAL SEQUENCE
   <220>
   <223> Degenerate Reverse Primer- GPPS 1
<400> 2

   tyaytttktt cttgtratga cgc 23
<210> 3
   <211> 22
   <212> DNA
   <213> ARTIFICIAL SEQUENCE
   <220>
   <223> Degenerate Forward Primer- GPPS 2
<400> 3
   tsgaratgat hcacacyatg tc 22
<210> 4
   <211> 23
   <212> DNA
   <213> ARTIFICIAL SEQUENCE
   <220>
   <223> Degenerate Reverse Primer 1- GPPS 2
   <220>
   <221> misc_feature
   <222> (3)..(3)
   <223> n is a, c, g, or t
<400> 4
   tanggaatrt aattmgcyar agc 23
<210> 5
   <211> 23
   <212> DNA

   <213> ARTIFICIAL SEQUENCE

   <220>
   <223> Degenerate Reverse Primer 2- GPPS 2
<400> 5
   ttyccwgcvg ttttccccar ttc 23
<210> 6

   <211> 25
   <212> DNA
   <213> ARTIFICIAL SEQUENCE
   <220>
   <223> Gene specific Forward Primer- GPPS 1
<400> 6
   agatgacgtt cttgatttca cgggc 25
<210> 7
   <211> 26
   <212> DNA
   <213> ARTIFICIAL SEQUENCE
   <220>
   <223> Gene specific Reverse Primer- GPPS 1
<400> 7
   ctttgagtta gatctaaaag tgcccg 26
<210> 8
   <211> 23
   <212> DNA
   <213> ARTIFICIAL SEQUENCE
   <220>
   <223> Gene specific Forward Primer- GPPS 2
<400> 8
   acgaccttcg tcggggaaaa ccg 23
<210> 9
   <211> 25
   <212> DNA
   <213> ARTIFICIAL SEQUENCE
   <220>
   <223> Gene specific Reverse Primer- GPPS 2
<400> 9
   gaccctcaat gccaatcgat ttcgc 25
<210> 10
   <211> 26
   <212> DNA
   <213> ARTIFICIAL SEQUENCE
   <220>
   <223> Terminal Primer Forward- GPPS1
<400> 10
   atgttatttt cttatggcct ttctcg 26
<210> 11
   <211> 26
   <212> DNA
   <213> ARTIFICIAL SEQUENCE
   <220>
   <223> Terminal Primer Reverse- GPPS1
<400> 11
   tttatttctt gtgatgactc tttgag 26
<210> 12
   <211> 20
   <212> DNA
   <213> ARTIFICIAL SEQUENCE
   <220>
   <223> Terminal Primer Forward- GPPS2
<400> 12
   atgccctttg tcgtgccaag 20
<210> 13
   <211> 28
   <212> DNA
   <213> ARTIFICIAL SEQUENCE
   <220>
   <223> Terminal Primer Reverse - GPPS2
<400> 13
   attttgccta taggcaatat aattagac 28
<210> 14
   <211> 1266
   <212> DNA
   <213> mangifera indica geranyl pyrophosphate synthase 1 (MiGPPS1), mRNA,
   complete cds
<400> 14
   atgttatttt cttatggcct ttctcggatt tcaataaatc ctagagcctc cttgttgact      60
   tgtcgttggc ttctctcgca tctgactggc tccctgagcc cttccacttc ttcacacact     120
   atcagtgact cagttcataa ggtttggggt tgcagagaag cttatacgtg gagtgttcct     180
   gccttgcatg gttttagaca ccaaattcat caccaaagca gctccctaat tgaggatcaa     240
   ctcgacccat tttcccttgt tgctgatgaa ctatcacttg ttgctaacag gctgcgctcc     300
   atggtagtca ctgaggtacc caagcttgcc tcagcagctg agtatttctt caaaatggga     360
   gtggagggaa agaggtttcg tcccgcggtt ttattgttga tggcaacagc cttgaatgtg     420
   catgtacttg agccacttcc tgaaggtgca ggagatgctt tgatgactga gctacgtaca     480
   agacaacaat gtatagctga gattactgag atgatccatg tagcaagcct tcttcacgat     540
   gatgtcttgg atgatgcaga tacaaggcgt ggcattggtt cgttaaattt agtaatgggg     600
   aataagttag ctgtattagc gggagatttt cttctatctc gcgcttgtgt tgcccttgct     660
   tcattgaaaa acacagaggt tgtatcatta ctggcaacag ttgtagagca tcttgttacc     720
   ggtgaaacaa tgcaaatgac tacttcatct gatcaacggt gtagcatgga atattatatg     780
   caaaaaacat actacaagac tgcttcattg atatcaaata gctgcaaggc aattgctctt     840
   cttgctgggc aatcagcaga agttgcaatg ttggcttttg agtttggaaa aaatctggga     900
   ctggcctacc aattaataga tgacgttctt gatttcacgg gcacatcagc ttcacttgga     960
   aagggatctt tatcggacat acggcatgga attgtaacgg ctcctatact gtttgcaatg    1020
   gaagaattcc cccagttgcg tgcagttatt gatcagggct ttgaaaatcc ttcaaacgtc    1080
   gatgtcgctc ttgaatacct tggcaagagt cggggaatac aaaggacgag agagctagcg    1140
   acaaaccatg ccaaccttgc tgcagctgcc atcgatgctc tacccaaaac tgacaatgaa    1200
   gaagtaagaa agtcaagacg ggcactttta gatctaactc aaagagtcat cacaagaaat    1260
   aaatga                                                               1266
<210> 15
   <211> 987
   <212> DNA
   <213> Mangifera indica geranyl pyrophosphate synthase 2 (MiGPPS2), mRNA,
   complete cds
<400> 15
   atgccctttg tcgtgccaag acgaaacaga tccctgtcag tttccgctgt tctcaccaaa      60
   gaagaaactc tcagggaaga agaggaagac ccaaaacccg tgttcgattt caagtcttat     120
   atgcttcaga aaggcaattc tgttaaccag gcccttgacg ccgtcgtttc aatccgtgaa     180
   cccaaaaaaa ttcacgaagc tatgaggtat tctcttctag cgggcggcaa gcgtgtaaga     240
   ccggtgctct gcatcgctgc gtgtgaactt gttggtggta atgagtccat ggcgatgccg     300
   gccgcttgtg ctgttgaaat gattcacacc atgtccttga ttcatgatga tctaccttgt     360
   atggataacg acgaccttcg tcggggaaaa ccgacaaacc acaaagtttt cggtgaagac     420
   gtcgccgttt tagccggtga tgcacttctt gccttttcgt ttgaaaacat ggctgtttct     480
   acggttggcg ttctgccttc gagggtggtc aaagcagttg gagaattagc gaaatcgatt     540
   ggcattgagg gtcttgttgc cggccaagtt gtggatataa actctgaagg tttaaaagaa     600
   gtgggcttag atcatcttga atttattcat cagcataaga cagctgcatt actggaagga     660
   tcagtcgttc ttggagcaat attgggtggt ggaagtgatg atgaagttga aaagctgaga     720
   acttttgctc ggtgtattgg gttgttgttt caggtggttg atgatattct tgatgtgaca     780
   aagtcatctc gggaacttgg aaagactgct ggcaaagatt tggtggctga taaagtcact     840
   tatcctaagt tgttggggat tgaaaaatca agggaattag ctgacaagtt aaataaagat     900
   gctcaacaac aattgtctgg ttttgatcag gagaaagccg cccctttgat tgctttgtct     960
   aattatattg cctataggca aaattga                                         987